(19) Europäisches Patentamt
European Patent Office
Office européen des brevets

(11) Veröffentlichungsnummer: **0 051 809**
**A1**

(12) **EUROPÄISCHE PATENTANMELDUNG**

(21) Anmeldenummer: **81109106.5**

(22) Anmeldetag: **28.10.81**

(51) Int. Cl.³: **C 07 D 487/04**, A 61 K 31/415
// (C07D487/04, 235/00, 235/00)

(30) Priorität: **12.11.80 DE 3042636**

(43) Veröffentlichungstag der Anmeldung: **19.05.82**
**Patentblatt 82/20**

(84) Benannte Vertragsstaaten: **AT BE CH DE FR IT LI LU NL SE**

(71) Anmelder: **C.H. BOEHRINGER SOHN, Postfach 200, D-6507 Ingelheim am Rhein (DE)**

(72) Erfinder: **Stähle, Helmut, Dr., Rotweinstrasse 23, D-6507 Ingelheim/Rhein (DE)**
Erfinder: **Köppe, Herbert, Dr., Neuweg 72, D-6507 Ingelheim/Rhein (DE)**
Erfinder: **Kummer, Werner, Dr., Georg-Scheuing-Strasse 15, D-6507 Ingelheim/Rhein (DE)**
Erfinder: **Kobinger, Walter, Prof. Dr., Belghofergasse 27, A-1120 Wien (AT)**
Erfinder: **Pichler, Ludwig, Dr., Gusshausstrasse 24, A-1040 Wien (AT)**
Erfinder: **Lillie, Christian, Dr. Mag., Hansi-Niese-Weg 12, A-1130 Wien (AT)**

(54) **Neue 7-(2,6-Dibrom-4-methylphenyl)-2,3-dihydro-imidazo(1,2-a)imidazole, deren Säureadditionssalze, diese enthaltende Arzneimittel und Verfahren zu deren Herstellung.**

(57) 7-(2,6-Dibrom-4-methylphenyl)-2,3-dihydro-imidazo[1,2-a]imidazole der allgemeinen Formel

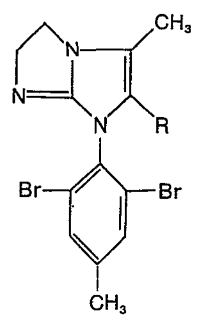

worin R ein Wasserstoffatom oder eine Methylgruppe bedeutet sowie deren Säureadditionssalze. Die Verbindungen können unter anderem durch Umsetzung von 2-(2,6-Dibrom-4-methylphenyl)-imino-imidazolidin mit einer Oxo-Verbindung der allgemeinen Formel

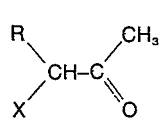

III

worin R wie oben angegeben definiert ist und X ein Chlor-, Brom- oder Jodatom bedeutet, hergestellt werden.

Die Verbindungen senken die Herzfrequenz und können als Mittel zur Behandlung von Coronarerkrankungen eingesetzt werden.

EP 0 051 809 A1

Die Erfindung betrifft neue substituierte 7-(2,6-Dibrom-4-methylphenyl)-2,3-dihydro-imidazo[1,2-a]imidazol-derivate der allgemeinen Formel I

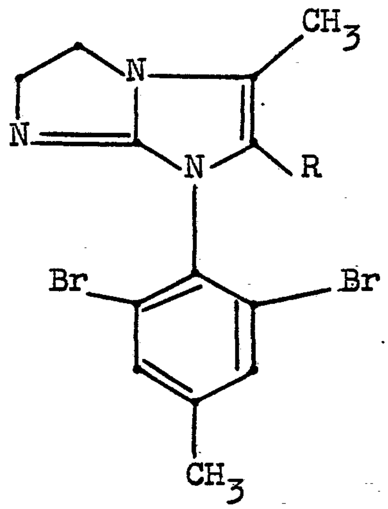

I

sowie deren physiologisch verträgliche Säureadditionssalze mit wertvollen therapeutischen Eigenschaften.

In der Formel I bedeutet R ein Wasserstoffatom oder eine Methylgruppe.

Die Herstellung der Verbindungen der Formel I kann nach verschiedenen Verfahren erfolgen, wobei sich die folgenden besonders bewährt haben:

a) Umsetzung von 2-(2,6-Dibrom-4-methylphenyl-imino)-imidazolidin der Formel

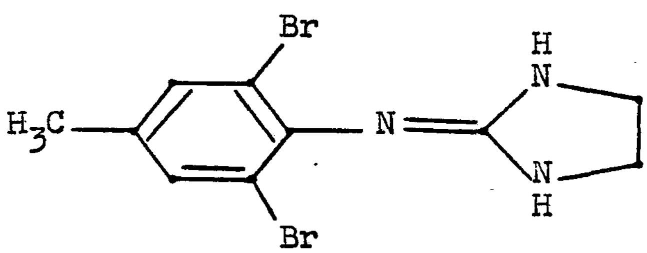

II

mit einer Oxo-Verbindung der allgemeinen Formel

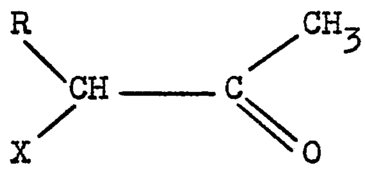

III

worin R wie oben angegeben definiert ist und X ein Chlor-, Brom- oder Jodatom bedeutet.

Die Umsetzung nach Verfahren a) erfolgt zweckmäßigerweise durch Erhitzen der Reaktionspartner - vorzugsweise in Gegenwart eines polaren oder unpolaren organischen Lösungsmittels - auf Temperaturen von etwa 60 - 180°C. Die speziellen Reaktionsbedingungen hängen im starken Maße von der Reaktivität der Umsetzungsteilnehmer ab. Die Umsetzung kann in Gegenwart eines säurebindenden Mittels wie z.B. Triäthylamin durchgeführt werden.

b) Cyclisierung eines 2-[N-Propargyl-N-(2,6-dibrom-4-methylphenyl)-amino]-imidazolins-(2) der allgemeinen Formel

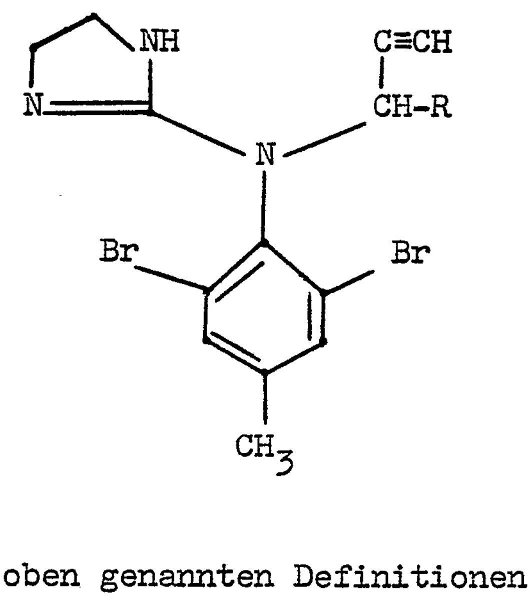

IV

worin R die oben genannten Definitionen besitzt.

0051809

Bei dem Verfahren b) wird ebenfalls bei erhöhter Temperatur - vorzugsweise zwischen 50 - 150°C - gearbeitet. Als Lösungsmittel kommen sowohl polare als auch unpolare Solventien in Frage. Es empfiehlt sich, die Umsetzung in Gegenwart einer organischen Base wie z.B. Trimethylbenzylammoniumhydroxid durchzuführen.

c) Wasserabspaltung aus einem 7-(2,6-Dibrom-4-methylphenyl)-5-hydroxy-5-methyl-2,3,5,6-tetrahydro-imidazo[1,2-a]-imidazol der allgemeinen Formel

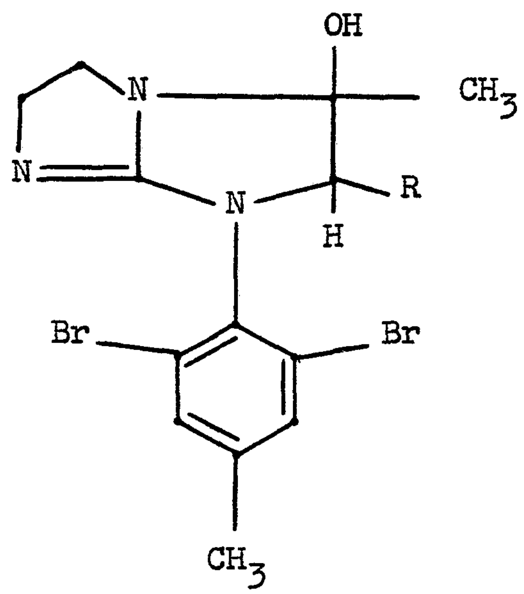

V

worin der Rest R die oben angegebenen Bedeutungen hat, bei erhöhter Temperatur und/oder in Gegenwart wasserabspaltender Mittel.

Die Wasserabspaltung nach Verfahren c),welche bei Temperaturen zwischen 60 - 180°C erfolgt,kann sowohl in Lösung als auch ohne Verwendung eines Lösungsmittels durchgeführt werden.

d) Umsetzung eines 1-(2,6-Dibrom-4-methylphenyl)-2-imino-4-methyl-imidazolins-(4) der allgemeinen Formel

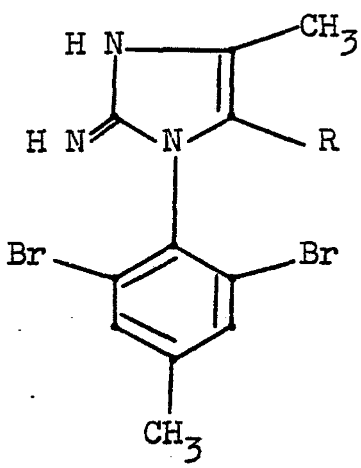

VI

worin R wie oben angegeben definiert ist, mit 1,2-Dibromäthan.

Die Synthese nach Verfahren d) wird ebenfalls bei erhöhter Temperatur - vorzugsweise bei 80 - 180°C - durchgeführt. Lösungsmittel können angewendet werden, sind jedoch nicht erforderlich.

Ausgangsverbindungen der Formel II sind z.B. in den belgischen Patentschriften Nr. 623 305, 687 657 und 705 944 beschrieben. Ausgangsverbindungen der Formel III sind handelsübliche Verbindungen und aus der Literatur bekannt. Ausgangsverbindungen der Formel IV sind z.B. in der deutschen Offenlegungsschrift 25 23 103 beschrieben worden.

Substanzen der Formel V können durch Umsetzung von 2-(2,6-Dibrom-4-methylphenyl-imino)-imidazolidin der Formel II mit Verbindungen der Formel III bei niederer Temperatur gewonnen werden. Ausgangsverbindungen der Formel VI erhält man bei der Umsetzung von N-(2,6-Dibrom-4-methylphenyl)-guanidin der Formel

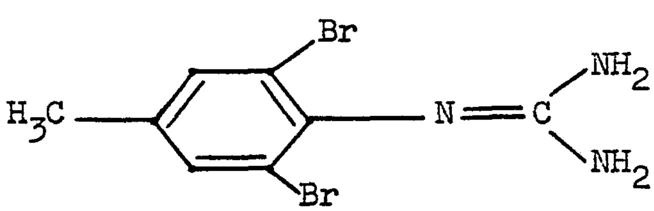

VII

mit Verbindungen der Formel III.

Die erfindungsgemäßen Imidazo[1,2-a]imidazole der allgemeinen Formel I können auf übliche Weise in ihre physiologisch verträglichen Säureadditionssalze überführt werden. Zur Salzbildung geeignete Säuren sind beispielsweise Mineralsäuren, wie Salzsäure, Bromwasserstoffsäure, Jodwasserstoffsäure, Fluorwasserstoffsäure, Schwefelsäure, Phosphorsäure, Salpetersäure oder organische Säuren wie Essigsäure, Propionsäure, Buttersäure, Capronsäure, Caprinsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, Äthanphosphonsäure, 8-Chlortheophyllin und dergleichen.

Die neuen Verbindungen der Formel I sowie deren Säureadditionssalze haben wertvolle pharmakologische Eigenschaften. Durch Untersuchungen an der Spinalratte konnte festgestellt werden, daß die Verbindungen eine starke herzfrequenzsenkende Wirkung besitzen. Aufgrund dieser bradykarden Wirkung kommen sie für die Behandlung von Coronarerkrankungen in Frage. Es wurde z.B. gefunden, daß die Verbindungen an der Spinalratte in einer Dosierung von 0,2 bis 1,2 mg/kg die Herzfrequenz um 150 Schläge je Minute zu senken.

Die Verbindungen der allgemeinen Formel I sowie deren Säureadditionssalze können enteral oder auch parenteral angewandt werden. Die Dosierung für die orale Anwendung liegt bei 0,1 bis 100 mg, vorzugsweise 0,5 bis 50 mg. Die Verbindungen der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen, Emulsionen oder Pulver; hierbei können zu deren Herstellung die üblicherweise verwendeten galenischen Hilfs- oder Trägerstoffe oder Spreng- oder Schmiermittel oder Substanzen zur Erzielung einer Depotwirkung Anwendung finden.

Die Herstellung solcher galenischer Darreichungsformen erfolgt auf übliche Weise nach den bekannten Fertigungsmethoden.

Die folgenden Beispiele erläutern die Erfindung ohne sie zu beschränken.

Beispiel 1

7-(2,6-Dibrom-4-methyl-phenyl)-2,3-dihydro-5-methyl-imidazo[1,2-a]-imidazol (Verfahren a).

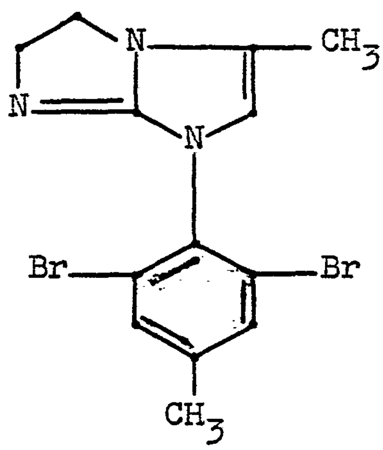

4,75 g 2-(2,6-Dibrom-4-methylphenylimino)imidazolidin (0,014 Mol) werden zusammen mit 1,4 ml (120%) Chloraceton in 25 ml Glykol-monomethyläther 12 Stunden lang am Rückfluß erhitzt. Die klare Reaktionsmischung wird hierauf im Vakuum zur Trockene eingeengt und das als Rückstand verbleibende, hellbraune Öl in 1N HCl gelöst. Die salzsaure Lösung wird mit verdünnter NaOH auf pH 7 eingestellt und ab diesem pH-Wert bei aufsteigenden pH-Werten (NaOH) fraktioniert mit Äther extrahiert. Die einheitlichen Ätherextrakte (Dünnschichtchromatogramm-Kontrolle) werden vereinigt, über $MgSO_4$ getrocknet und im Vakuum eingeengt. Das neue Imidazo[1,2-a]imidazol fällt zunächst ölig an, um nach kurzer Zeit durchzukristallisieren. Ausbeute: 0,95 g entsprechend 17,9 % der Theorie. Schmp.: 144-146°C.

$C_{13}H_{13}Br_2N_3$ (371,1)

| | | | |
|---|---|---|---|
| C(Gef)42,05 | H(Gef)3,18 | Br(Gef)42,21 | N(Gef)10,90 |
| C(Ber)42,08 | H(Ber)3,53 | Br(Ber)43,07 | N(Ber)11,32 |

Beispiel 2

7-(2,6-Dibrom-4-methylphenyl)-2,3-dihydro-5,6-dimethyl-imidazo-[1,2-a]-imidazol (Verfahren a)

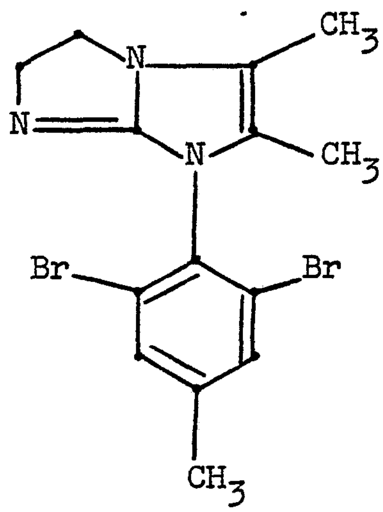

5,0 g (0,015 Mol) 2-(2,6-Dibrom-4-methylphenylimino)-imidazolidin werden zusammen mit 1,8 g (110 %) 96%-igem 3-Chlor-2-butanon in 25 ml Glykol-monomethyläther etwa 30 Stunden am Rückfluß erhitzt. Die Reaktionsmischung wird sodann im Vakuum zur Trockene eingeengt und das im Rückstand verbleibende Öl in 1 N Salzsäure gelöst. Die salzsaure Lösung wird mit verdünnter NaOH auf einen pH-Wert von 7 eingestellt und ab diesem pH-Wert bei steigenden pH-Werten (NaOH) fraktioniert mit Äther extrahiert. Die dünnschichtchromatographisch einheitlichen Ätherextrakte werden vereinigt, über Magnesiumsulfat getrocknet und der Äther im Vakuum abgezogen. Das resultierende Öl kristallisiert nach kurzer Zeit durch. Ausbeute: 0,4 g entsprechend 7% der Theorie. Schmp.: 154-156°C.

$C_{14}H_{15}Br_2N_3$ (385,1)

| | | | |
|---|---|---|---|
| C(Gef)43,80 | H(Gef)3,89 | Br(Gef)41,68 | N(Gef) 10,9 |
| C(Ber)43,66 | H(Ber)3,93 | Br(Ber)41,50 | N(Ber) 10,9 |

## Formulierungsbeispiele

### Beispiel A: Dragées

| | | |
|---|---|---|
| Wirkstoff gemäß Erfindung | | 5 mg |
| Milchzucker | | 65 mg |
| Maisstärke | | 130 mg |
| sek. Calciumphosphat | | 40 mg |
| lösliche Stärke | | 3 mg |
| Magnesiumstearat | | 3 mg |
| kolloidale Kieselsäure | | 4 mg |
| | insgesamt | 250 mg |

Herstellung:

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpreßt, die dann in üblicher Weise mit Hilfe von Zucker, Talkum und Gummi arabicum dragiert werden.

### Beispiel B: Ampullen

| | | |
|---|---|---|
| Wirkstoff gemäß Erfindung | | 1,0 mg |
| Natriumchlorid | | 18,0 mg |
| dest. Wasser | ad | 2,0 mg |

Herstellung:

Wirkstoff und Natriumchlorid werden in Wasser gelöst und unter Stickstoff in Glasampullen abgefüllt.

Beispiel C:Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

## Patentansprüche:

1. 7-(2,6-Dibrom-4-methylphenyl)-2,3-dihydro-imidazo[1,2-a]imidazole der allgemeinen Formel

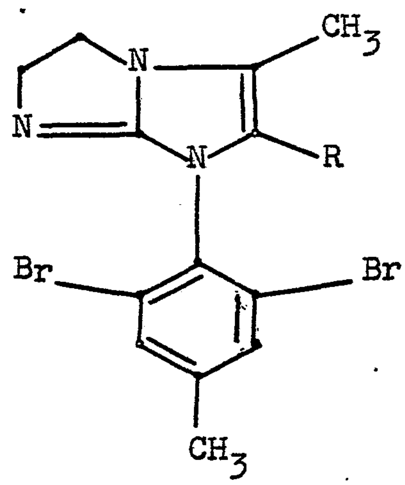

I

worin R ein Wasserstoffatom oder eine Methylgruppe bedeutet, sowie deren Säureadditionssalze.

2. Verfahren zur Herstellung von Verbindungen der allgemeinen Formel I nach Anspruch 1, dadurch gekennzeichnet, daß man

a) 2-(2,6-Dibrom-4-methylphenyl)-imino-imidazolidin der Formel

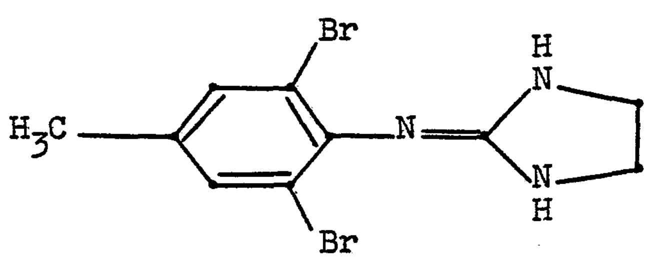

II

mit einer Oxo-Verbindung der allgemeinen Formel

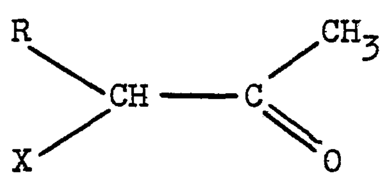

III

worin R wie oben definiert ist und X ein Chlor-, Brom- oder Jodatom bedeutet, umsetzt; oder

b) ein 2-[N-Propargyl-N-(2,6-dibrom-4-methylphenyl)-amino]-imidazolin-(2) der allgemeinen Formel

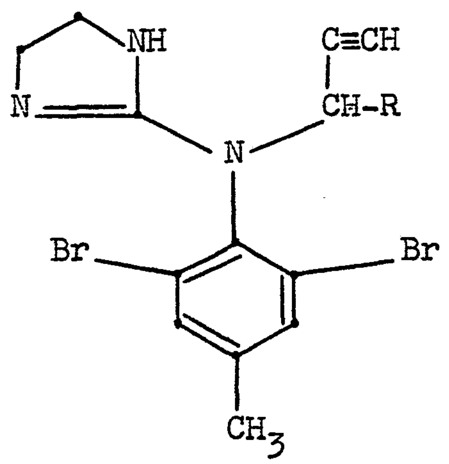

IV

worin R die oben genannten Definitionen besitzt, cyclisiert; oder

c) aus einem 7-(2,6-Dibrom-4-methylphenyl)-5-hydroxy-5-methyl-2,3,5,6-tetrahydro-imidazo-[1,2-a]imidazol der allgemeinen Formel

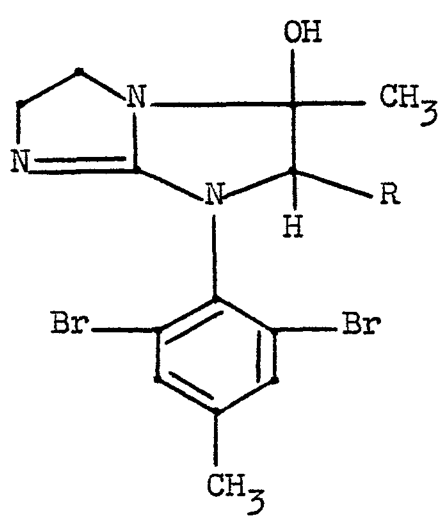

V

worin R die oben angegebenen Bedeutungen hat, bei erhöhter Temperatur und/oder in Gegenwart wasserabspaltender Mittel

0051809

Wasser abspaltet; oder

d) ein 1-(2,6-Dibrom-4-methylphenyl)-2-imino-4-methyl-imidazolin-(4) der allgemeinen Formel

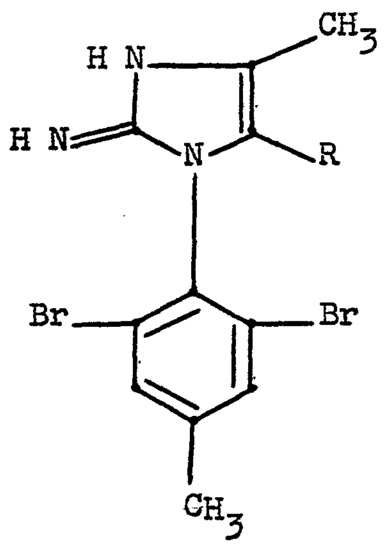

VI

worin R wie oben angegeben definiert ist, mit 1,2-Dibromäthan umsetzt und die erhaltene Verbindung gewünschtenfalls in ein Säureadditionssalz überführt.

3. Pharmazeutische Zubereitungen, dadurch gekennzeichnet, daß sie eine oder mehrere Verbindungen nach Anspruch 1 als Wirkstoff enthalten.

4. Verfahren zur Herstellung von pharmazeutischen Zubereitungen nach Anspruch 4, dadurch gekennzeichnet, daß man eine oder mehrere Verbindungen nach Anspruch 1 mit üblichen galenischen Hilfs- oder Trägerstoffen oder Spreng- oder Schmiermitteln oder Substanzen zur Erzielung einer Depotwirkung verarbeitet.

5. Verwendung von Verbindungen nach Anspruch 1 bei der Behandlung von Coronarerkrankungen.

6. Methode zur Behandlung von Coronarerkrankungen, dadurch gekennzeichnet, daß man eine oder mehrere Verbindungen nach Anspruch 1 in Form von Zubereitungen nach Anspruch 4 verabreicht.

0051809

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 81 10 9106.5

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| X | EP - A1 - 0 006 451 (C.H. BOEHRINGER SOHN)<br>* Ansprüche 1 bis 6 *<br>-- | 1-5 | C 07 D 487/04<br>A 61 K 31/415<br>// /C 07 D 487/04<br>235/00, 235/00) |
| A | DE - A - 2 118 261 (C.H. BOEHRINGER SOHN)<br>* Ansprüche 2,6,7 *<br>-- | 2-4 | |
| A | DE - A - 2 361 188 (JANSSEN PHARMACEUTICA)<br>---- | | RECHERCHIERTE SACHGEBIETE (Int. Cl.3)<br>A 61 K 31/415<br>C 07 D 487/04 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5

Unvollständig recherchierte Patentansprüche: --

Nicht recherchierte Patentansprüche: 6

Grund für die Beschränkung der Recherche:

Verfahren zur therapeutischen Behandlung des menschlichen oder tierischen Körpers

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Berlin | 27-01-1982 | FROELICH |